# FASCICULE DE BREVET EUROPEEN

(11) **EP 1 833 741 B1**
(45) Date de publication et mention de la délivrance du brevet: **30.07.2008**
(21) Numéro de dépôt: 05850567.8
(22) Date de dépôt: 21.12.2005
(51) Int. Cl.: B65D 83/08

(54) **DISPOSITIF DE STOCKAGE ET DE DISTRIBUTION A L'UNITE D'OBJETS SE PRESENTANT SOUS FORME DE FEUILLES OU BANDELETTES MINCES**
VORRICHTUNG ZUR ABLAGE UND ABGABE VON EINZELOBJEKTEN IN FORM VON FOLIEN ODER DÜNNEN STREIFEN
DEVICE FOR STORING AND DISPENSING IN SINGLE-UNITS OBJECTS IN THE FORM OF SHEETS OR THIN STRIPS

(30) Priorité: 21.12.2004 FR 0413639
(43) Date de publication de la demande: 19.09.2007
(73) Titulaire: AIRSEC (SOCIETE PAR ACTIONS SIMPLIFIEE), 94600 Choisy Le Roi (FR)
(72) Inventeur: NOBILET, Roger, 77500 Chelles (FR); PORTIER, Benoît, 18000 Bourges (FR)
(74) Mandataire: Schweighart, Peter
(86) Numéro de dépôt international: PCT/FR2005/003221
(87) Numéro de publication internationale: WO 2006/067334

(56) Documents cités:
- EP-A- 1 250 877
- WO-A-94/14682
- WO-A-20/04024593
- DE-A1- 4 205 805
- FR-A- 2 709 475
- GB-A- 1 573 084
- GB-A- 2 210 603
- US-A- 3 517 855
- US-A- 4 071 165
- US-A1- 2003 106 900

## Description

### Domaine de l'invention

L'invention concerne un dispositif de stockage et de distribution à l'unité d'objets singularisés allant du rigide au souple, objets se présentant sous la forme de feuilles, plaquettes, bandelettes ou lamelles minces et le conteneur d'objets singularisés équipé d'un tel dispositif de distribution.

De nombreux objets ayant la forme de feuilles, plaquettes, bandelettes ou lamelles minces sont utilisés comme consommables. De tels objets sont notamment des « strip » tests en forme de bandelettes ou lamelles rigides utilisées par exemple à des fins de diagnostic ou de contrôle dans le domaine médical. D'autres objets à distribuer ayant une forme carrée ou rectangulaire plus ou moins souples, peuvent également se présenter sous la forme de feuille mince, soit seuls ou avec leur emballage protecteur, par exemple des pansements, des patchs transdermiques ou des produits alimentaires tels que des chewing-gums ou autres.

Pour des raisons évidentes, en particulier d'hygiène, mais aussi afin d'éviter toute dégradation et ainsi améliorer la durée de conservation des objets, ceux-ci sont placés à l'abri des pollutions extérieures et/ou des attaques physico-chimiques résultant du niveau d'humidité relative, de la lumière, en particulier des rayons UV et autres substances chimiques ou encore des dégradations par effet mécanique.

Pour des raisons également d'hygiène, de conservation et de protection de nombreux dispositifs de stockage et de distribution ont été imaginés afin de permettre une distribution unité par unité des objets se présentant sous forme de feuilles, plaquettes, bandelettes ou lamelles minces, de manière à distribuer le nombre exact d'objets requis et éviter ainsi toute pollution due à une sortie involontaire d'objets.

Lorsque ces objets sont déjà préemballés, la seule fonction à remplir par ces dispositifs de stockage et de distribution est celle de contenir un nombre déterminé d'objets et de pouvoir les distribuer à l'unité sans devoir ouvrir une boite constituant la forme la plus classique d'emballage.

De tels dispositifs de distribution d'objets doivent être simples, peu onéreux à produire et d'une utilisation aisée.

### Etat de la technique

De nombreux dispositifs de stockage et de distribution d'objets de forme sensiblement plate sont décrits dans la littérature technique, en particulier dans celle constituée par les demandes de brevet et/ou brevets publiés.

Le plus simple des systèmes de stockage et de distribution spécifiquement adaptés à des feuilles ou lamelles minces opère par l'intermédiaire de la combinaison d'une pression exercée de bas en haut sur le stock de feuilles empilées et d'une ouverture partielle d'un couvercle libérant une partie des feuilles à extraire, l'extraction une a une des feuilles se faisant manuellement par un doigt de l'utilisateur, qui fait glisser la feuille supérieure sur le paquet de feuilles se trouvant dans le dispositif de distribution.

Un tel dispositif augmente le risque de contamination. Au moment de la préhension d'une feuille ou par exemple d'une lamelle test, les doigts de l'utilisateur viennent au contact de cette lamelle ou de la lamelle immédiatement adjacente, au risque de dégrader ou de contaminer par pollution externe, les surfaces actives de ces feuilles ou lamelles.

Selon un premier document de l'art antérieur (US 4,240, 564), est décrit un dispositif de stockage et de distribution à l'unité de feuilles de savon.

Le dispositif de stockage et de distribution apparaît être composé d'un conteneur, d'un couvercle avec une fente de distribution, d'un dispositif à ressort permettant de maintenir en appui sur la face inférieure du couvercle, les feuilles empilées les unes sur les autres, d'un système d'éjection venant prendre appui sur la feuille de savon à distribuer et actionné par un bouton poussoir situé sur le face supérieure du couvercle.

Chaque feuille est en partie translatée vers l'extérieur du conteneur par l'intermédiaire du système d'éjection, de manière à en permettre la préhension par l'utilisateur.

Ce dispositif de distribution possède de nombreux désavantages techniques dont l'un par exemple, est le fait qu'il semble incapable de distribuer des objets se présentant sous la forme de feuilles très minces et/ou souples; car dans ce cas, plusieurs feuilles peuvent simultanément être poussées hors du conteneur.

En outre, ce dispositif est également complexe et relativement coûteux à produire car il requiert l'usage d'un dispositif d'extraction des feuilles par l'intermédiaire d'un système coulissant équipé d'une lame glissante de faible épaisseur venant s'appuyer sur la feuille à distribuer, d'un système de ressort pour maintenir les feuilles en appui les unes sur les autres et d'un couvercle à cinématique complexe.

Enfin, si plusieurs feuilles sont extraites simultanément, sans pouvoir en contrôler le nombre, il y a un certain risque de les détériorer lorsqu'elles sont réintégrées dans le conteneur rendant encore plus aléatoire le système de distribution.

Aucun des dispositifs issus de ou similaires à ceux de l'art antérieur ne donne de résultats satisfaisants car ces dispositifs ne permettent pas de contrôler le nombre d'objets feuilles extraits du conteneur lorsque celles ci sont de très fine épaisseur. En outre, de tels dispositifs ne permettent pas de garantir l'absence de pollution ou de salissures des feuilles inopinément extraites puis réintroduites dans le conteneur car non utilisées en raison de leur surnombre, détériorant ainsi la qualité du stockage des feuilles.

Un autre document (FR n° 2 709 475) décrit un distributeur de cartes dont la sortie de cartes est réalisée au moyen d'un ergot-poussoir, coulissant dans une lumière longitudinale du couvercle du distributeur. Ledit ergot-poussoir est solidaire d'une lame à effet mémoire élastique, qui est dans un état quasi-linéaire, à rayon de courbure pratiquement infini lorsque l'empilement des cartes est maximal, et prend la forme d'un arc de cercle avec un rayon de courbure de plus en plus court et orienté vers le fond du réceptacle quand l'empilement des cartes disparaît. L'extrémité de cette lame est pourvue d'un embout à fort coefficient de frottement, en contact avec la pile de cartes.
Ainsi, la lame munie d'un embout à fort coefficient de frottement, est composée de deux matériaux, et a la forme d'un arc de cercle plus ou moins prononcé selon l'épaisseur de la pile de cartes.

Un autre document (GB n° 2 210 603) décrit un distributeur de cartes, dont la sortie est réalisée au moyen d'un élément mobile flexible d'entraînement, qui se compose d'une lame-ressort et d'un embout en caoutchouc, solidaire de ladite lame. Cet élément mobile flexible d'entraînement est actionné par un ergot mobile coulissant dans une lumière-guide du couvercle, selon un mouvement horizontal longitudinal. Ladite lame-ressort et l'embout en caoutchouc à laquelle il est lié, sont tous les deux en appui sur la pile de cartes, et assurent l'extraction d'une carte à la demande.
Il peut être relevé que l'élément mobile flexible d'entraînement est réalisé au moyen de deux matériaux, dont l'un est en caoutchouc. Cet élément mobile flexible d'entraînement, à savoir la lame-ressort associée à un embout en caoutchouc, est dans une position arc-boutée entre la pile de cartes et la face interne du couvercle, l'incurvation de la lame-ressort étant dirigée dans la direction opposée au sens de sortie de cartes.

Un autre document (WO 2004/024593) décrit un distributeur de minces feuillets, dont l'extraction à l'unité est réalisée au moyen d'un éjecteur, formé par un couvercle mobile longitudinalement, d'une partie souple en forme de demi-sphère creuse solidaire dudit couvercle mobile, et d'un appendice interne à et solidaire de ladite demi-sphère creuse. Pour provoquer la distribution d'un feuillet, l'utilisateur agit par pression sur l'éjecteur, dont la demi-sphère creuse, sous l'action de cette pression, s'écrase et repousse verticalement l'appendice. L'extrémité avale de cet appendice arrive au contact de la pile de feuillets, s'y déforme en prenant une forme en "L" en direction de la sortie , entraînant ainsi un feuillet, grâce à un mouvement de translation du couvercle, qui simultanément referme le container.
Originellement vertical, il apparaît que l'élément mobile d'entraînement, n'est pas en contact permanent avec l'objet à entraîner, qu'il prend la forme de la lettre "L", dès lors qu'il est en contact avec ledit objet, et qu'il exige deux mouvements simultanés pour extraire le feuillet, l'un vertical et l'autre horizontal.

Enfin, un dernier document (US n° 3 517 855) décrit un système de distribution complexe mettant en oeuvre de multiples pièces mécaniques assemblées. Un déplacement dans le sens vertical descendant d'un bouton poussoir est converti en un déplacement horizontal grâce à un système d'engrenage et de crémaillère. Un ressort permet le retour en arrière du bouton-poussoir par l'exercice d'une force de rappel dans le sens vertical ascendant.
Il s'agit dans ce document d'un système mécanique complexe et non d'un élément mobile déformable.

Ainsi, l'état de la technique révèle que l'élément mobile flexible d'entraînement peut être, soit constitué de plusieurs types de matériaux, soit formé d'un système mécanique complexe. Mais ces dispositifs de distribution d'objets, apparaissent de conception complexe, de réalisation coûteuse, de manipulation difficile quand les feuillets à extraire sont de faible épaisseur et souples.

### Objet de l'invention

Le problème posé est de réaliser un dispositif de distribution d'objets se présentant sous la forme de feuilles, plaquettes, bandelettes ou lamelles minces qui peuvent aussi bien être rigide que semi-rigide jusqu'à souple, à géométrie plane en particulier sensiblement carrée, rectangulaire, polygonale, circulaire, elliptique, qui permet non seulement de distribuer ces objets unité par unité, mais encore de résoudre tout ou partie des inconvénients précédemment évoqués.

En effet, de tels objets, de part leur géométrie (faible épaisseur) et leur propriétés mécaniques (module de rigidité) ne peuvent être distribués par les dispositifs décrits dans l'état de la technique, c'est à dire par des dispositifs poussant de tels objets par la tranche arrière pour en assurer le déplacement.

Par faible épaisseur, on entend des objets dont l'épaisseur est inférieure à 2 mm, préférentiellement inférieure à 1 mm et très préférentiellement inférieure 0,5 mm.

Ces objets se présentant sous la forme d'un empilement organisé à l'intérieur d'un conteneur de stockage et ont capacité à glisser les uns sur les autres. Ils présentent un coefficient de frottement statique entre la surface supérieure de l'un et la surface inférieure de l'autre qui lui est immédiatement adjacent, constant et connu et de relativement faible valeur.

On entend par coefficient de frottement statique la force initialement mesurée pour déplacer horizontalement un corps d'un poids donné revêtu d'une surface particulière sur une autre surface, alors que le coefficient de frottement dynamique correspond à la mesure de cette même force lorsque le mouvement de glissement s'est enclenché. Ces coefficients varient en fonction des types de matériaux et des états de surface mais également en fonction des températures, des temps durant lesquels les deux matériaux ont été en contact et de la pression exercé par le corps mobile sur la surface plane de glissement. Le coefficient de frottement dynamique est en général inférieur au coefficient de frottement statique.

Un tel dispositif de distribution peut être adapté pour pouvoir être manoeuvré d'une seule main, facilitant ainsi sa manipulation, l'autre main étant libre pour assurer la préhension de l'objet distribué. Toutefois dans le cas où il y aurait présence d'un dispositif de sécurité enfant cette disposition ne s'applique pas, car les deux mains sont nécessaires à la manipulation.

### Sommaire de l'invention

Dès lors, l'invention concerné un dispositif de stockage et de distribution, de type boîtier, pour objets se présentant sous la forme de feuilles, plaquettes, bandelettes ou lamelles minces aussi bien rigide que semi-rigide à flexible, à géométrie plane en particulier sensiblement carrée, rectangulaire, polygonale, circulaire, elliptique, permettant la distribution unité par unité desdits objets à distribuer, comportant:
- un moyen de stockage de type conteneur avec couvercle, contenant les objets à distribuer, stockés empilés les uns sur les autres sur une surface de stockage, ledit moyen de stockage comprenant une surface de guidage des objets à distribuer et une ouverture de distribution permettant le passage unité par unité des objets à distribuer,
- et un moyen de distribution
caractérisé en ce que
ledit moyen de distribution est constitué par un élément flexible, déformable, mobile d'entraînement, dont le contact de l'une de ses extrémités avec la face supérieure de l'objet à distribuer est en contact transversal surface à surface selon au moins une ligne tangentielle, de coefficient de frottement statique supérieur au coefficient de frottement statique existant entre deux surfaces en regard de deux objets contigus à distribuer, et dont la coupe longitudinale est en "S" ou en presque "U".

Un moyen de distribution en forme de "S" est décrit dans le document EP-A-1250877, mais le contact de ce moyen de distribution avec les objets à distribuer n'est pas de type surface à surface.

Un autre objet de l'invention est l'utilisation du dispositif de stockage et de distribution pour la distribution d'objets en forme de feuilles, plaquettes, bandelettes ou lamelles minces tels que des « strip » tests en forme de bandelettes ou lamelles rigides utilisées par exemple à des fins de diagnostic ou de contrôle dans le domaine médical ou d'objets à distribuer de forme carré ou rectangulaire pouvant également se présenter sous la forme de feuille mince, soit seuls ou avec leur emballage protecteur, en particulier des pansements, des patchs transdermiques, des produits alimentaires en particulier des chewing-gums ou autres. Ainsi, ce dispositif de distribution d'objets s'utilise pour séparer et distribuer unité par unité un stock d'objets à distribuer se trouvant stockés empilés en attente d'utilisation et protégés du milieu extérieur.

Le moyen de stockage de type conteneur, ci-après dénommé conteneur comporte un fond formé par la combinaison de deux surfaces contiguës, l'une étant la surface de stockage et l'autre étant la surface de guidage.

La surface de stockage, constituant une surface d'appui sur laquelle les objets à distribuer sont empilés, est une surface généralement plane qui peut être inclinée, formant alors un angle aigu avec le plan constitué par le couvercle lorsque prolongé en direction de l'ouverture de distribution ou parallèle au plan du couvercle ; la surface de stockage est contiguë à la surface de guidage. Cette surface de stockage est préférentiellement inclinée afin de pré-orienter les objets à distribuer par rapport à la surface de guidage.

La surface de guidage est préférentiellement une surface curviligne développable, constituant une rampe de sortie débouchant sur l'ouverture de distribution, et est plus particulièrement une portion de cylindre dont le plan de tangence au niveau du raccordement de la surface de guidage avec l'ouverture de distribution forme un angle, avec le plan constitué par le couvercle au niveau de l'ouverture, compris entre 10° et 90° et préférentiellement entre 45° et 60°.

La surface de guidage peut également être constituée par un ou plusieurs plans inclinés par rapport au plan formé par le couvercle au niveau de l'ouverture de distribution, de manière à former une trémie permettant de guider l'objet à distribuer. Lorsque la surface de guidage forme un seul dièdre ayant une arête vive constitué par la fente de distribution, l'angle d'inclinaison de la surface de guidage par rapport au plan constitué par le couvercle et passant par la fente de distribution est alors généralement comprise entre 20° et 60° et préférentiellement de 45°.

Les surfaces de guidage peuvent avoir toutes autres formes permettant la fonction de guidage des objets en direction de la fente de distribution.

Afin de favoriser la fonction de guidage, les surfaces de guidage sont préférentiellement constituées de matériaux rigides qui permettent le glissement aisé des objets à distribuer. Les surfaces de guidage des objets à distribuer peuvent également être traitées, par exemple par grainage, de manière à ce que le coefficient de frottement mesuré entre la surface inférieure de l'objet à distribuer et la face intérieure du conteneur permette également au dernier objet à distribuer de glisser sur la surface interne du conteneur.

Le conteneur comporte également deux parois verticales sensiblement parallèles permettant de clore partiellement le conteneur et de positionner les objets à distribuer latéralement. Le conteneur comporte également une troisième paroi verticale sensiblement perpendiculaire aux deux parois latérales, suivant des considérations d'esthétique du conteneur-distributeur, positionnée sur le coté opposé à l'ouverture de distribution sur lequel les objets à distribuer viennent prendre appui par leur tranche.

Selon un autre mode de réalisation, les deux parois permettant de clore partiellement le conteneur et de positionner les objets à distribuer latéralement sont concourantes.

Par ailleurs, la troisième paroi, perpendiculaire aux deux parois latérales, peut soit former un angle droit avec le plan du couvercle, soit former un angle aigu ou obtus avec le plan du couvercle.

Un tel conteneur sera avantageusement réalisé par toute technique d'injection ou de thermoformage, à partir de matériaux thermoplastiques présentant une certaine rigidité.

Le conteneur est clos sur sa partie supérieure par un couvercle:
- qui peut être fixe et est alors équipé d'une lumière de glissement afin de permettre à l'élément flexible, déformable, mobile d'entraînement de l'objet à distribuer d'être déplacé en translation à l'intérieur de cette lumière de guidage
- qui peut être mobile en rotation ou en translation par rapport au conteneur.

Dans le cas où le couvercle est mobile en rotation, il peut être articulé en totalité ou en partie suivant un axe positionné sur le conteneur.
✔ Quand le couvercle est articulé en totalité, l'axe de rotation de type par exemple charnière intégrale peut être placé sur une des extrémité du conteneur, coté ouverture de distribution.
Quand le couvercle est articulé en partie, c'est dire qu'une partie du couvercle est fixe par rapport au conteneur, et que l'autre partie est en rotation par rapport à ce conteneur, l'axe de rotation de type par exemple charnière intégrale peut être placé en un lieu compris entre les deux extrémités du couvercle.

Dans le cas d'un couvercle mobile en rotation, l'objet à extraire du conteneur peut l'être à l'une ou l'autre des extrémités du dit conteneur.

Un système de rappel de la partie mobile du couvercle en rotation peut être présent sous la forme d'un moyen approprié tel que par exemple un ressort ou une lame déformable intégré ou autres.

Dans le cas d'un couvercle mobile en translation, il peut éventuellement comporter sur sa surface d'extrémité supérieure un bossage permettant à l'utilisateur de faire coulisser le couvercle par rapport au conteneur.

Un système de rappel en position initiale du couvercle mobile en translation, tel que par exemple un ressort de rappel, peut éventuellement être présent entre le couvercle et le conteneur.

Le couvercle, qu'il soit fixe ou mobile , peut comporter éventuellement une nervure sur sa face interne afin de maintenir l'élément flexible, déformable, mobile d'entraînement en contact avec la face supérieure de l'objet à distribuer. L'élément flexible, déformable, mobile d'entraînement peut rester également toujours maintenu en contact avec la face supérieure de l'objet à distribuer grâce à son poids propre, en particulier en l'absence de nervure.

Dans le cas d'un couvercle fixe, il peut être assemblé sur le conteneur par toute technique d'assemblage rapide tel qu'un clipsage ou par tous autres moyens d'assemblage, tel que par exemple un soudage. Dans le cas d'un couvercle mobile, le moyen d'assemblage rapide tel que clipsage ou autres, est associé à un système de guidage par rapport au conteneur.

Un système de clipsage et de coulissage par l'intermédiaire de rails avec butée permet de solidariser le couvercle et le conteneur sans pour autant restreindre la capacité du couvercle de translater sur le conteneur.

Le couvercle est préférentiellement réalisé en matériau polymère rigide moulé par injection ou par thermoformage.

Les matériaux polymères souhaitables pour réaliser le conteneur et le couvercle sont généralement et non exclusivement choisis dans le groupe de matériaux composés de polymères thermoplastiques, comprenant en particulier les polyoléfines telles que les polyéthylènes, les polypropylènes, les copolymères d'éthylène/propylène et leurs mélanges, les polyamides (PA), les polystyrènes (PS), les copolymères d'acrylonitrile-butadiène-styrène (ABS), les copolymères de styrène-acrylonitrilé (SAN), les polyméthacrylates de méthyl (PMMA), les polyéthylènetéréphtalates (PET), les polybutylènetéréphtalates (PBT), les polyacétals (POM), les polychlorures de vinyle (PVC), les polycarbonates (PC).

Le conteneur et le couvercle peuvent également recevoir, entre ledit conteneur et ledit couvercle, un témoin d'ouverture du conteneur avant la première ouverture. Ce moyen d'inviolabilité ou encore témoin d'inviolabilité, est formée, par exemple, de micro-liens reliant tout ou partie de la surface périphérique inférieure du couvercle à tout ou partie de la surface périphérique supérieure de par exemple une bague ou un collier ou tout système d'accrochage venant fixer le couvercle sur le conteneur. Ces micro-liens sont des micro-points de liaison, indépendants les uns des autres mais forment un pourtour
ou une simple zone dentée de liaison entre le couvercle mobile et la bague ou collier ou système fixant le couvercle au conteneur ou entre l'élément flexible mobile d'entraînement et le conteneur ou le couvercle solidaire.

Ces micro-points sont rompus lors de la première ouverture par l'application d'une force d'arrache exercée sur le couvercle ou sur l'élément flexible, déformable, mobile d'entraînement dans le sens horizontal.

L'élément flexible, déformable, mobile d'entraînement est fait d'au moins un matériau souple et est susceptible de flexion sans déformation permanente. Il peut avoir la forme d'un 'S' ou d'un presque 'U' lorsque visualisé en coupe longitudinale. Il peut avoir diverses formes telles que par exemple:
- celle d'un doigt de section circulaire, elliptique ou polygonale, dont l'extrémité avale est munie d'une barre transversale perpendiculairement à son axe longitudinal
- ou celle d'une lame mince.

La longueur de ladite barre transversale ou la largeur de ladite lame mince peuvent être au plus égale à celle du conteneur.

Quand l'élément flexible, déformable, mobile d'entraînement est une lame mince, la largeur de la lame mince peut être constante sur toute la longueur de l'élément flexible ou bien subir un rétrécissement régulier ou irrégulier.

Préférentiellement, les formes de l'élément flexible sont rectangulaire, triangulaire, ou en forme de "T".
- Quand l'élément flexible est un rectangle, sa largeur donnant le contact tangentiel transversal est au plus égale à une valeur très légèrement inférieure à la largeur L du conteneur,
- Quand l'élément flexible est un triangle, la grande base du triangle constitue au moins la ligne de contact tangentielle transversale avec l'objet à extraire,
- Quand l'élément flexible a la forme de la lettre "T", la barre transversale du "T" constitue au moins la ligne de contact tangentielle transversale avec l'objet à distribuer, et cette barre a une longueur au plus égale à une valeur très légèrement inférieure à la largeur L du conteneur.

Cet élément flexible, déformable, mobile d'entraînement a pour caractéristique d'être en contact permanent avec l'objet à entraîner lors du déplacement, selon au moins une ligne de contact tangentielle transversale par rapport à l'axe longitudinal de sortie dudit objet, et d'avoir ainsi capacité à translater l'objet à distribuer, tout en restant appuyé sur sa surface.

Selon l'invention, le contact tangentiel transversal par rapport à l'axe longitudinal de sortie de l'objet s'étend à une surface de contact selon le mode "surface à surface" avec l'objet à distribuer.

Ainsi, la ligne de contact tangentielle transversale ou la surface de contact tangentielle transversale de type "surface à surface" avec l'objet à distribuer par rapport à l'axe longitudinal de sortie de l'objet, ont une longueur comprise entre 0,25 fois la largeur L du conteneur et une longueur très légèrement inférieure à la largeur L du conteneur. Ladite longueur est préférentiellement comprise entre 0,6 L et 1 L.

La surface de l'élément flexible, déformable, mobile d'entraînement a également pour caractéristique de présenter un coefficient de frottement statique, lorsque déterminé par rapport à la face supérieure de l'objet à entraîner, supérieur au coefficient de frottement statique existant entre les deux surfaces en regard des deux objets contigus (n) et (n-1) à distribuer. Il peut être ou non solidaire du couvercle selon que celui ci est mobile ou pas, c'est à dire, l'élément flexible, déformable, mobile d'entraînement est solidaire du couvercle quand ledit couvercle est mobile et est désolidarisé du couvercle quand ledit couvercle est fixe.

Dans le cas d'un couvercle mobile en rotation par rapport au conteneur, l'élément flexible, déformable, mobile d'entraînement est solidaire de la partie mobile du couvercle.

Dans le cas d'un couvercle mobile en translation par rapport au conteneur, l'élément flexible déformable, mobile d'entraînement est solidaire du couvercle mobile.

Lorsque l'élément flexible, déformable, mobile d'entraînement est désolidarisé du couvercle, alors, il est équipé d'un sabot coulissant dans la lumière du couvercle fixe, le sabot étant déplacé manuellement par un mouvement de va et vient du doigt de l'utilisateur.

L'élément flexible, déformable, mobile d'entraînement peut être réalisé avec au moins une matière issue du groupe des matières présentant les caractéristiques propres aux compositions élastomériques, c'est à dire présentant un module de rigidité proche de ceux des élastomères, une capacité à recouvrance de la forme initiale sans hystérésis, un coefficient de frottement supérieur à celui en général obtenu sur des thermoplastiques rigides, et une dureté 'shore' également inférieure à celles considérées pour des thermoplastiques rigides.

L'élément flexible, déformable, mobile d'entraînement peut être mono-matériau, c'est à dire formé d'une seule matière thermoplastique élastomérique formulée, sous la forme d'une composition adaptée en terme de propriétés mécaniques et de propriétés de coefficient de frottement, mettant en oeuvre au moins un élastomère thermoplastique et un autre composé thermoplastique rigide, préférentiellement de type oléfinique ou être multi-matériaux, c'est à dire comprendre une âme faite d'un type de matériau et une peau faite d'un autre type de matériau de type élastomérique, afin de séparer les propriétés mécaniques des propriétés de surface, et donc pouvoir éventuellement être obtenue par d'autres techniques telles que le revêtement. Les zones en matériaux élastiques peuvent être alors formées d'au moins un élastomère thermoplastique d'origine naturelle ou synthétique. Le ou les élastomères mis en oeuvre peuvent être choisis préférentiellement dans le groupe constitué par des élastomères de type caoutchoucs naturels, caoutchouc synthétique, en particulier les caoutchoucs de mono-oléfines, tels que, par exemple, les polymères d'isobutylène/isoprène, éthylène-acétate de vinyle (EVA), éthylène-propylène (EPR), éthylène-propylène-diène (EPDM), éthylène-esters acryliques (EMA-EEA), les polymères fluorés, les caoutchouc de dioléfines, tels que, par exemple, les polybutadiène, les copolymères de butadiène-styrène (SBR), les caoutchoucs à base de produits de condensation tels que, par exemple, les caoutchoucs thermoplastiques polyesters et polyuréthanes, les silicones, les caoutchoucs styréniques, tels que styrène-butadiène-styrène(SBS), les styrène-isoprène-styrène (SIS), les styrene-ethylène butadiene styrene (SEBS) et autres copolymères bloc, mis en oeuvre seuls ou en mélange, formulés ou non.

Le mode de réalisation peut être également celui de l'injection thermoplastiques ou de l'extrusion et découpe de feuille par toute technique d'extrusion ou de calandrage.

Cependant et dans un mode de réalisation préférentielle, lorsque l'élément flexible, déformable, mobile d'entraînement est solidaire du couvercle mobile, il est alors préférable de considérer les techniques d'injection bi-matière pour réaliser en une seule opération l'ensemble constitué par l'élément flexible, déformable, mobile d'entraînement et le couvercle. Le couvercle est réalisé alors en thermoplastique rigide, et l'élément flexible mobile d'entraînement en composition formulée à partir d'élastomère thermoplastiques associés à par exemple des polyoléfines.

Lorsque l'élément flexible, déformable, mobile d'entraînement est désolidarisé du couvercle fixe et possède un sabot de guidage, alors le sabot est en matériau rigide et l'obtention d'une telle pièce se fait par injection bi-matière ; il peut être également en mono matière formulée afin d'obtenir un compromis entre le besoin de coulisser du sabot dans sa rainure et le besoin de coefficient de frottement adéquate par rapport à l'objet à distribuer qu'il faut entraîner.

L'ouverture de distribution permettant le passage unité par unité des objets à distribuer est une ouverture préférentiellement de type fente dont les bords forment des lèvres ouvertes, ayant éventuellement capacité à s'occulter grâce à un volet flexible, dans le but de rendre l'ensemble du dispositif raisonnablement étanche, sans pour autant qu'une force de pression.trop importante soit nécessaire pour l'ouvrir.

La géométrie des lèvres de l'ouverture de distribution peut être telle qu'elle favorise la sortie de l'objet à distribuer, c'est-à-dire qu'elle présente la forme d'un chanfrein, d'un biseau ou d'un décalage de l'une des lèvres par rapport à l'autre.

L'ouverture de distribution a préférentiellement une forme de fente avec des dimensions qui permettent le passage d'un seul objet à la fois. Toutefois lorsque le couvercle est reculé pour être ensuite refermé, l'ouverture de distribution peut être constituer par ce vide entre conteneur et couvercle en voie de fermeture. Lorsque l'ouverture de distribution est de type fente, sa largeur et son épaisseur sont généralement légèrement supérieure à la largeur et à l'épaisseur de l'objet à distribuer.

L'ouverture sous forme de fente peut être partie intégrante du conteneur, partie intégrante du couvercle ou se former à l'intersection des deux parties du conteneur-distributeur, conteneur et couvercle, lors de leur assemblage.

Lorsqu'un volet flexible de faible épaisseur vient occulter la fente de distribution, celui ci sera avantageusement formé en matériaux élastomériques moulés par les techniques d'injection bi-matière en même temps que peut l'être l'élément flexible, déformable, mobile d'entraînement.

Un système sécuritaire d'ouverture à l'égard des enfants peut éventuellement être associé au dispositif de stockage et de distribution à l'unité d'objets.

Ledit système sécuritaire d'ouverture à l'égard des enfants, en vue de leur rendre l'ouverture du bouchon-couvercle difficile voire impossible, peut être installé au droit du couvercle. Ce dispositif peut être un bouton poussoir faisant partie intégrante du couvercle, et permettant par la combinaison d'une pression verticale maintenue et d'une translation simultanée, de déverrouiller le système sécuritaire et d'assurer le fonctionnement du distributeur. Le bouton poussoir est en parfait alignement avec le couvercle lorsque ledit bouton est en position verrouillée, et est légèrement en saillie par rapport à la surface d'extrémité supérieure du couvercle lorsqu'il est en position déverrouillée.

Dans le cas de la présence d'un système sécuritaire d'ouverture à l'égard des enfants, l'utilisateur, par une pression verticale exercée sur le bouton pressoir de ce système le déverrouille. Le système peut également comporter des micro-points sécables témoin de première ouverture positionnés entre le bouton poussoir et son logement.

Les produits particulièrement sensibles à l'humidité ambiante sont nombreux et peuvent être plus précisément par exemple des matériaux médicamenteux qui peuvent également se présenter sous forme de bandes pour tests ou autres.

Le dispositif pourra alors comporter un conteneur et/ou un couvercle et/ou un élément flexible, déformable, mobile d'entraînement dessicatifs dans la mesure ou une certaine étanchéité pour le stockage de produits sensibles à l'humidité ambiante est possible lorsque l'ouverture de distribution à capacité à être refermée, soit par l'intermédiaire d'un volet soit lorsque clos par un bouchon rendu solidaire ou non du dispositif.

Ces conteneurs sont rendus dessicatifs par l'un au moins des moyens qui consistent en un revêtement interne du fond du conteneur et/ou de la surface interne de la paroi du conteneur et/ou du couvercle au moyen d'une composition polymère thermoplastique dessicative, en l'introduction d'un insert dans ledit conteneur-distributeur, en le revêtement de l'élément flexible, déformable, mobile d'entraînement au moyen d'une composition élastomérique dessicative, et/ou en l'introduction d'un matériau dessicatif placé dans un logement particulier sur la surface interne du conteneur et/ou du couvercle.

Tous ces moyens dessicatifs sont implantés séparément ou simultanément pour augmenter l'efficacité de leur action déshydratante par un effet de masse.

Lors de l'opération de distribution, par un mouvement de translation par rapport au conteneur exercé sur élément flexible mobile d'entraînement, soit directement, si le couvercle est fixe, soit par l'intermédiaire du couvercle mobile lorsque ledit moyen flexible est solidaire du couvercle, va permettre à l'objet (n) qui se trouve au sommet de la pile de venir glisser sur l'objet positionner juste en dessous (n-1), l'objet (n) ainsi sélectionné étant guidé jusqu'à la fente de sortie grâce à la surface de guidage constitué par le fond curviligne du conteneur. L'objet sélectionné (n) ainsi guidé est poussé partiellement à l'extérieur de la fente pour permettre sa préhension par l'utilisateur.

Par l'inversion du sens de translation de l'élément flexible, déformable, mobile d'entraînement, celui ci reprend sa position initiale, mais ne peut déplacer l'objet (n-1) car celui ci vient en buté sur la paroi du conteneur opposée au coté sur lequel la fente de distribution se situe. Le coefficient dynamique de frottement entre l'élément flexible mobile d'entraînement et la face supérieure de l'objet (n-1) est suffisamment faible pour ne pas provoquer un risque de déformation de l'objet (n-1) lorsque celui ci vient en buté sur la paroi du conteneur.

D'autres avantages du distributeur d'objets se présentant sous la forme de feuilles, plaquettes, bandes ou bandelettes, lamelles, très minces, rigides, semi rigides ou souples, selon l'invention, apparaîtront à la lecture de l'exemple de réalisation détaillé de l'invention, en se référant aux dessins donnés à titre d'illustration, dans lequel :
- La.figure 1 représente une vue en perspective d'un dispositif de stockage et de distribution équipé de son dispositif de distribution ;
- La figure 2 représente une vue en coupe d'un dispositif de stockage et de distribution dans sa position au repos ;
- La figure 3 représente une vue en coupe d'un dispositif de stockage et de distribution dans sa position au ouverte ;
- La figure 4 représente une vue en coupe d'un dispositif de stockage et de distribution dans sa position à nouveau fermée après translation de l'objet à distribuer.
- La figure 5 représente une vue en perspective d'un dispositif de stockage et de distribution suivant un autre mode de réalisation.
- La figure 6 représente une vue en perspective d'un dispositif de stockage et de distribution suivant un autre mode de réalisation.
- La figure 7 représente une vue en coupe d'un dispositif de stockage et de distribution selon le mode de réalisation de la figure 6 dans sa position de repos.
- La figure 8 représente une vue en coupe d'un dispositif de stockage et de distribution selon le mode de réalisation de la figure 6 dans sa position actionnée.
- La figure 9 représente une vue en perspective du dispositif de stockage et de distribution selon un autre mode de réalisation.
- La figure 10 représente une vue en coupe du dispositif de stockage et de distribution selon le mode de réalisation de la figure 9 dans sa position actionnée.

### Description détaillée de l'invention

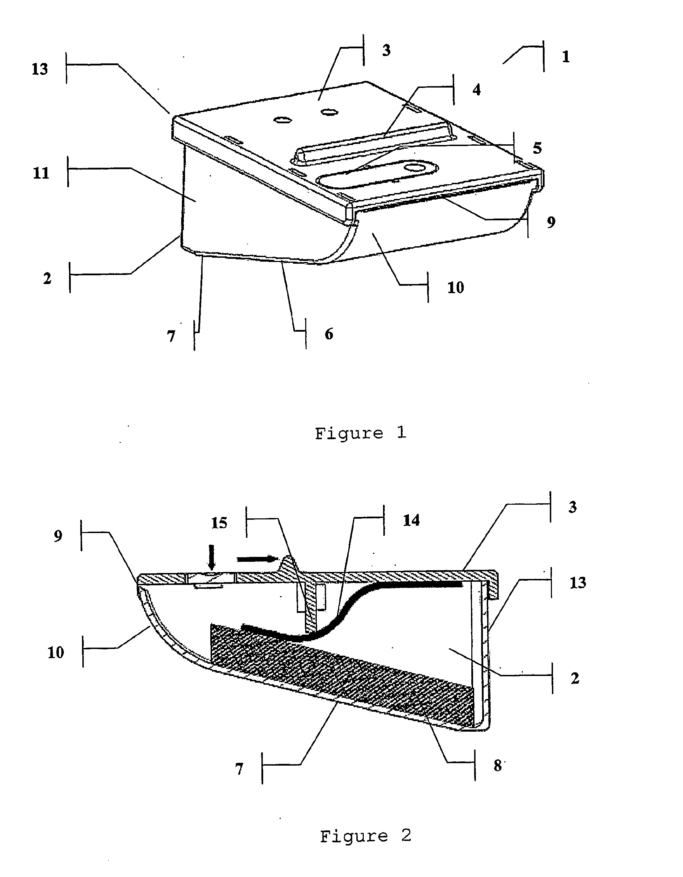

La figure 1 représente une vue en perspective du dispositif de stockage et de distribution (1) comprenant un conteneur (2), un couvercle (3) équipé sur sa surface d'extrémité supérieure d'un bossage (4) permettant à l'utilisateur de faire coulisser le couvercle, et d'un système sécuritaire d'ouverture à l'égard des enfants (5).

Ce dispositif de stockage et de distribution (1) est composé d'un moyen de stockage de type conteneur ouvert (2) comportant un fond (6) constitué par une surface plane inclinée de stockage (7) interne au conteneur sur laquelle les objets à distribuer (8) sont empilés, et par une surface dite de guidage (10) toujours interne au conteneur et contiguë à la surface de stockage (7). Cette surface de stockage (7) constituant une surface d'appui pour les objets à distribuer (8) est incliné de manière à pré-orienter les objets à distribuer par rapport à la surface de guidage curviligne développable (10), constituant en quelque sorte une rampe de sortie.

Le conteneur comporte également deux parois verticales parallèles (11) et (12) permettant de clore partiellement le conteneur et de positionner les objets (8) latéralement. Le conteneur comporte également une troisième paroi verticale (13) perpendiculaire au deux premières, positionnée sur le coté opposé à l'ouverture de distribution (9) en forme de fente sur lequel les objets à distribuer (8) viennent prendre appui par leur tranche.

Comme mentionné précédemment, les objets à distribuer (8) sont, dans ce cas descriptif particulier, des lamelles de tests fréquemment utilisées dans le domaine des diagnostics médicaux ou analyses chimiques.

Les figures 2 à 4 représentent les différentes étapes aboutissant à l'extraction d'une lamelle.mince.

Selon l'invention, la figure 2 représente une vue en coupe du dispositif de stockage et de distribution dans sa position au repos ;
le dispositif de stockage et de distribution (1) comprenant un conteneur (2) contenant les objets (8) à distribuer par la fente de distribution (9), un couvercle (3) et un élément flexible, déformable, mobile d'entraînement (14) solidaire du couvercle (2).

Le dispositif de stockage et de distribution (1) comporte une nervure (15) sur la face interne du couvercle afin de maintenir l'élément flexible, déformable, mobile d'entraînement (14) en contact avec la face supérieure de l'objet à distribuer (8).

L'élément flexible, déformable, mobile d'entraînement (14) est constitué d'une languette faite d'un matériau flexible et est susceptible de flexion sans déformation permanente. Il a la forme d'un 'S' lorsque visualisée en coupe. Cet élément flexible mobile d'entraînement (14) a pour caractéristique d'être en contact avec l'objet à entraîner (8), et d'avoir capacité à translation tout en restant appuyé sur la surface de l'objet à distribuer. Il a également pour caractéristique de posséder une surface présentant un coefficient de frottement statique, lorsque déterminé par rapport à la face supérieure de l'objet à entraîner, supérieur au coefficient de frottement statique existant entre les deux surfaces en regard des deux objets contigus (n) et (n-1) à distribuer. Il est solidaire du couvercle mobile.

La surface de stockage constituant une surface d'appui sur laquelle les objets à distribuer sont empilés (7), est une surface plane inclinée et contiguë à une surface dite de guidage préférentiellement curviligne développable (10). Cette surface de stockage est inclinée afin de pré-orienter les objets à distribuer par rapport à la surface de guidage préférentiellement curviligne développable (10), elle-même constituant en quelque sorte une rampe de sortie pour les objets à distribuer (8)

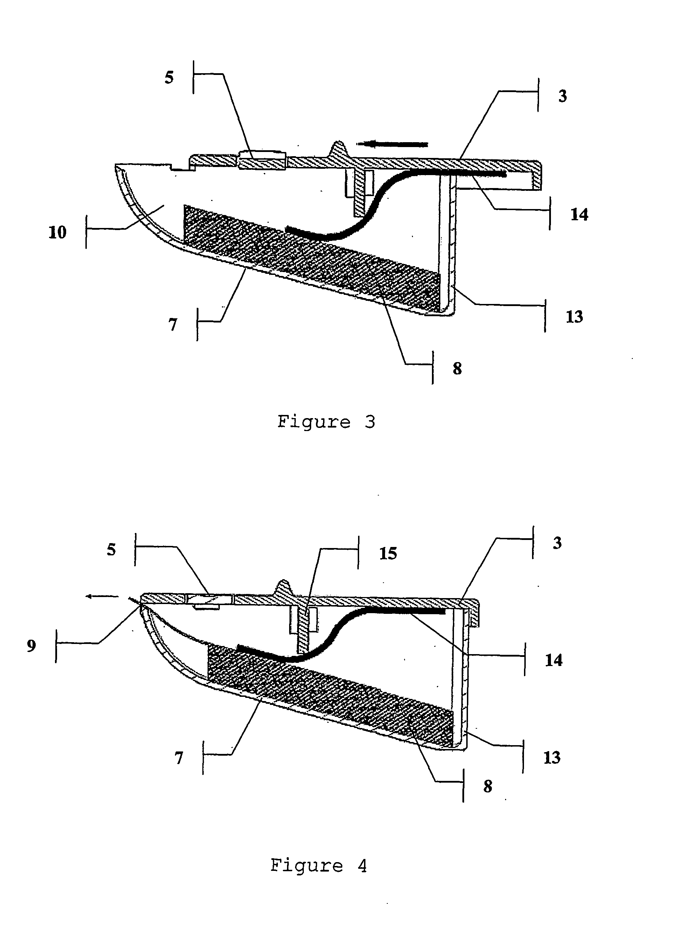

La figure 3 représente une vue en coupe d'un dispositif de stockage et de distribution (1) dans sa position ouverte, après déverrouillage du système sécuritaire d'ouverture à l'égard des enfants. Le couvercle (3) est alors translaté dans la direction opposée au coté où est situé la fente de distribution en entraînant l'élément flexible, déformable, mobile d'entraînement (14) qui lui est solidaire, l'élément flexible, déformable, mobile d'entraînement (14) restant toujours en contact avec la face supérieure de l'objet à distribuer (8) grâce à la présence d'une nervure (15) située sur la face interne du couvercle.

La figure 4 représente une vue en coupe d'un dispositif de stockage et de distribution (1) dans sa position à nouveau fermée après translation de l'objet à distribuer. Ainsi, par inversion du sens de la translation exercée sur le couvercle (3) solidaire de l'élément flexible, déformable, mobile d'entraînement (14), le couvercle (3) reprend sa position initiale fermée et l'élément flexible mobile d'entraînement (14) entraîne l'objet à distribuer (n) vers la fente de distribution (9). Lorsque l'élément flexible mobile d'entraînement (14) arrive en fin de course, l'objet à distribuer sélectionné (n) est poussé partiellement à l'extérieur de la fente (9) pour permettre sa préhension par l'utilisateur.

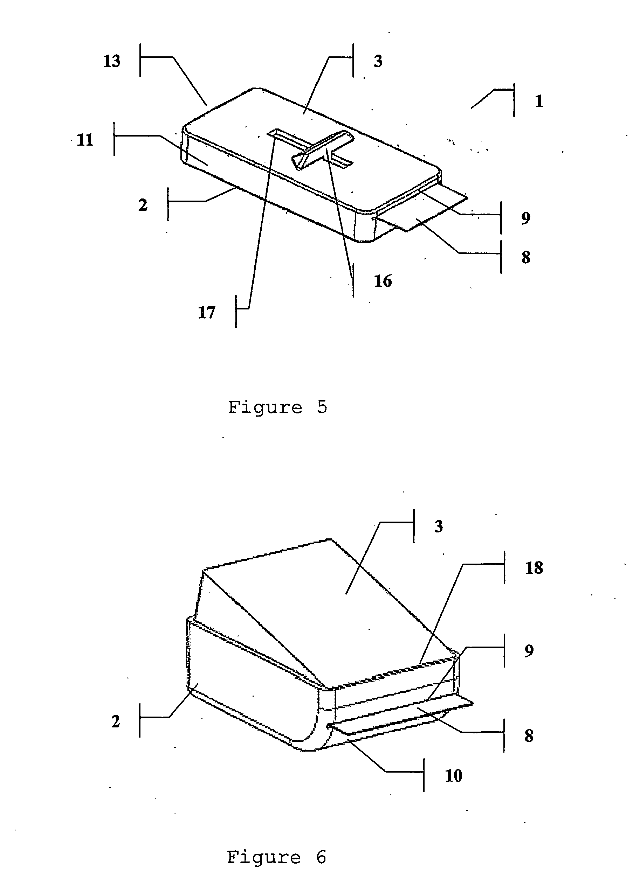

La figure 5 représente une vue en perspective du dispositif de stockage et de distribution (1) comprenant un conteneur (2), un couvercle (3) équipé sur sa surface supérieure d'un sabot à bouton de préhension (16) coulissant dans la lumière (17) et permettant à l'utilisateur de translater 1 élément flexible déformable mobile d'entraînement. Le conteneur (2) comporte une surface de guidage (10) non représentée à l'intérieur, sa forme externe étant parallélépipédique.

La figure 6 représente une vue en perspective du dispositif de stockage et de distribution (1) comprenant un conteneur (2), un couvercle (3) articulé suivant un axe (18) positionné sur une des extrémités du conteneur (2), coté ouverture de distribution (9), cet axe de rotation étant par exemple de type charnière intégrale.

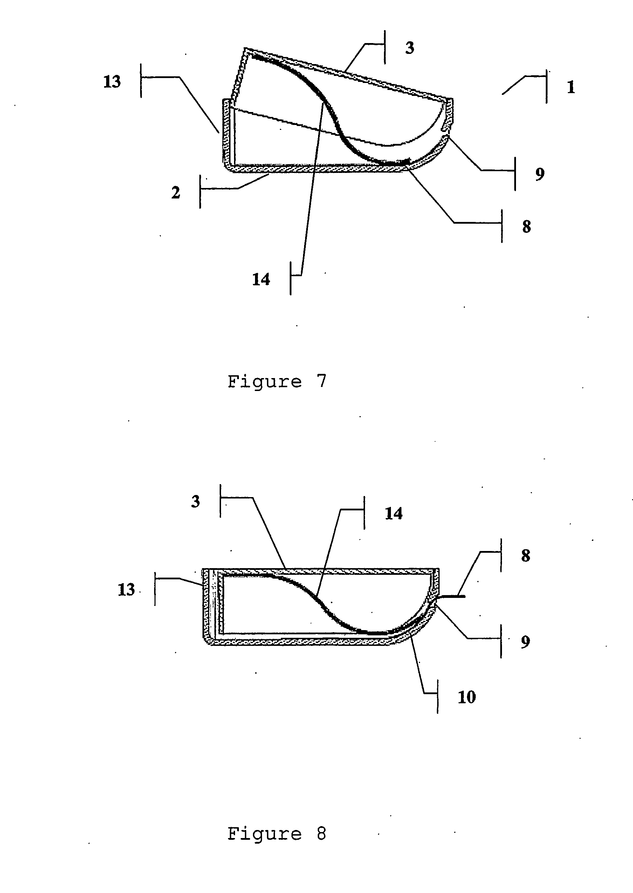

La figure 7 représente une vue en coupe du dispositif de stockage et de distribution (1) selon le mode de réalisation de la figure 6 dans sa position de repos.

La figure 8 représente une vue en coupe du dispositif de stockage et de distribution (1) selon le mode de réalisation de la figure 6 dans sa position actionnée. Selon ce mode de réalisation, le couvercle (3) est articulé en totalité, l'axe de rotation (18) de type par exemple charnière intégrale étant placé sur une des extrémités du conteneur (2), coté ouverture de distribution (9).

Ainsi, une pression exercée par la main de l'utilisateur sur le couvercle (3) articulé, du coté opposé au coté comportant l'ouverture de distribution (9), permet de déplacer l'élément flexible, déformable, mobile d'entraînement (14) et d'entraîner l'objet à distribuer (8) en direction de l'ouverture de distribution (9), ladite ouverture de distribution (9) de type fente présentant aussi elle-même une forme biseautée facilitant le guidage de l'objet à distribuer (8) vers la sortie.

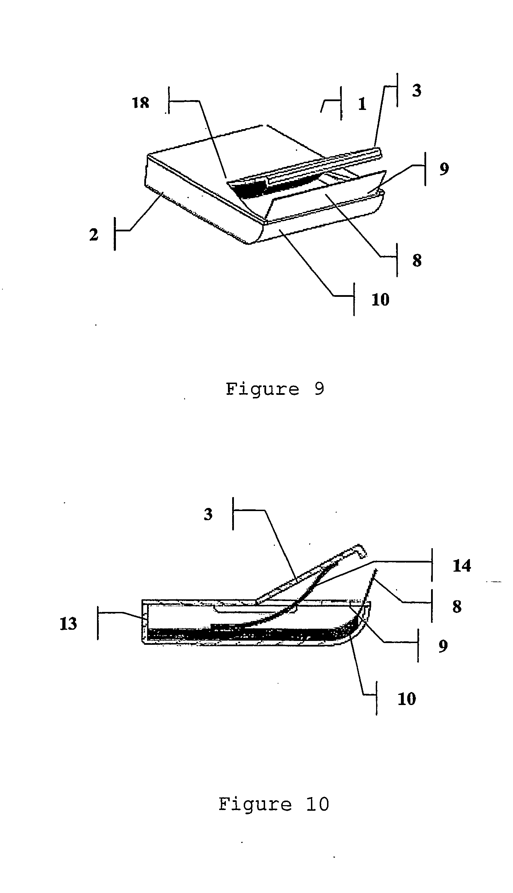

La figure 9 représente une vue en perspective du dispositif de stockage et de distribution (1) selon un autre mode de réalisation. Selon cet autre mode de réalisation, le couvercle n'est articulé qu'en partie, c'est dire qu'une partie du couvercle (3) est fixe par rapport au conteneur (2), et que l'autre partie est en rotation par rapport à ce conteneur(2), l'axe de rotation (18) de type par exemple charnière intégrale étant placé en un lieu compris entre les deux extrémités du couvercle.

La figure 10 représente une vue en coupe du dispositif de stockage et de distribution(1) selon le mode de réalisation de la figure 9 dans sa position actionnée. L'utilisateur ouvre la partie mobile du couvercle (3) en le déplaçant en rotation vers le haut, libérant ainsi l'ouverture de distribution (9) du conteneur (2). Le déplacement en rotation de la partie mobile du couvercle (3), solidaire de l'élément flexible, déformable, mobile d'entraînement (14), va permettre à l'objet (8) situé au sommet de la pile d'être tiré et guidé jusqu'à l'ouverture de sortie (9).

## Revendications

1. Dispositif de stockage et de distribution (1), de type boîtier, pour objets se présentant sous la forme de feuilles, plaquettes, bandelettes ou lamelles minces, à géométrie plane en particulier sensiblement carrée, rectangulaire, polygonale, circulaire, elliptique, permettant la distribution unité par unité desdits objets à distribuer(8), comportant:
- un moyen de stockage de type conteneur (2) avec couvercle (3), contenant les objets à distribuer (8), stockés empilés les uns sur les autres sur une surface de stockage (7), ledit moyen de stockage comprenant une surface de guidage (10) des objets à distribuer (8) et une ouverture de distribution (9) permettant le passage unité par unité des objets à distribuer (8),
- et un moyen de distribution
**caractérisé en ce que** ledit moyen de distribution est constitué par un élément flexible, déformable, mobile d'entraînement (14), dont le contact de l'une de ses extrémités avec la face supérieure de l'objet à distribuer (8) est un contact transversal surface à surface selon au moins une ligne tangentielle, de coefficient de frottement statique supérieur au coefficient de frottement statique existant entre deux surfaces en regard de deux objets contigus à distribuer (8), et dont la coupe longitudinale est en "S" ou.en presque "U".

2. Dispositif de stockage et de distribution (1) selon la revendication 1, **caractérisé en ce que** l'élément flexible, déformable, mobile d'entraînement (14) a la forme d'un doigt de section circulaire, elliptique ou polygonale, dont l'extrémité avale est munie d'une barre transversale perpendiculairement à son axe longitudinal, assurant le contact transversal surface à surface selon au moins une ligne tangentielle avec l'objet à distribuer (8).

3. Dispositif de stockage et de distribution (1) selon la revendication 2, **caractérisé en ce que** l'élément flexible, déformable, mobile d'entraînement (14) est une lame mince, de largeur constante sur toute la longueur dudit élément flexible, ou subissant un rétrécissement régulier ou irrégulier.

4. Dispositif de stockage et de distribution (1) selon l'une quelconque des revendications 1 à 3, **caractérisé en ce que** l'élément flexible, déformable, mobile d'entraînement (14) a préférentiellement une forme triangulaire, rectangulaire ou en forme de "T".

5. Dispositif de stockage et de distribution (1) selon la revendication 4, **caractérisé en ce que** lorsque l'élément flexible, déformable, mobile d'entraînement (14) a une forme rectangulaire, sa largeur est au plus égale à une valeur très légèrement inférieure à la largeur L du conteneur.

6. Dispositif de stockage et de distribution (1) selon la revendication 4, **caractérisé en ce que** lorsque l'élément flexible, déformable, mobile d'entraînement (14) a une forme triangulaire, la grande base du triangle constitue le contact transversal avec l'objet à extraire.

7. Dispositif de stockage et de distribution (1) selon la revendication 4, **caractérisé en ce que** lorsque l'élément flexible, déformable, mobile d'entraînement (14) a une forme de "T", la barre transversale du "T" constitue le contact transversal avec l'objet à distribuer, et cette barre a une longueur au plus égale à une valeur très légèrement inférieure à la largeur L du conteneur.

8. Dispositif de stockage et de distribution (1) selon l'une quelconque des revendications 1 à 7, **caractérisé en ce que** la surface de contact transversal avec l'objet à distribuer, a une longueur comprise entre 0,25 fois la largeur L du conteneur et une longueur très légèrement inférieure à ladite largeur L du conteneur.

9. Dispositif de stockage et de distribution (1) selon la revendication 8, **caractérisé en ce que** la longueur est préférentiellement comprise entre 0,6 L et une longueur très légèrement inférieure à ladite largeur L du conteneur.

10. Dispositif de stockage et de distribution (1) selon l'une quelconque des revendications 1 à 9, **caractérisé en ce que** le moyen de stockage de type conteneur (2) comporte un fond (6) constitué par la combinaison de deux surfaces contiguës, l'une étant la surface de stockage (7) et l'autre étant la surface de guidage (10).

11. Dispositif de stockage et de distribution (1) selon l'une quelconque des revendications 1 à 10, **caractérisé en ce que** la surface de stockage (7), constituant une surface d'appui sur laquelle les objets à distribuer (8) sont empilés, est une surface généralement plane inclinée formant un angle aigu avec le plan constitué par le couvercle (3) et est contiguë à la surface de guidage (10).

12. Dispositif de stockage et de distribution (1) selon l'une quelconque des revendications 1 à 10, **caractérisé en ce que** la surface de stockage (7), constituant une surface d'appui sur laquelle les objets à distribuer (8) sont empilés, est une surface parallèle au plan du couvercle (3) et est contiguë à la surface de guidage (10).

13. Dispositif de stockage et de distribution (1) selon l'une quelconque des revendications 1 à 12, **caractérisé en ce que** la surface de guidage (10) est une surface curviligne développable, constituant une rampe de sortie et débouchant sur l'ouverture de distribution (9).

14. Dispositif de stockage et de distribution (1) selon l'une quelconque des revendications 1 à 12 **caractérisé en ce que** la surface de guidage (10) est constituée par un ou plusieurs plans inclinés par rapport au plan formé par le couvercle (2) au niveau de l'ouverture de distribution (9), de manière à former une trémie permettant de guider l'objet à distribuer.

15. Dispositif de stockage et de distribution (1) selon l'une quelconque des revendications 1 à 14, **caractérisé en ce que** le conteneur (2) comporte deux parois verticales sensiblement parallèles (11) et (12) permettant de clore partiellement le conteneur (2) et de positionner les objets à distribuer (8) latéralement et également une troisième paroi verticale (13) sensiblement perpendiculaire aux deux parois verticales (11) et (12), positionnée sur le coté opposé à l'ouverture de distribution (9) sur lequel les objets à distribuer (8) viennent prendre appui par leur tranche.

16. Dispositif de stockage et de distribution (1) selon l'une quelconque des revendications 1 à 14, **caractérisé en ce que** les deux parois (11) et (12) sont concourantes.

17. Dispositif de stockage et de distribution (1) selon l'une quelconque des revendications 1 à 16, **caractérisé en ce que** le conteneur (2) est clos sur sa partie supérieure par un couvercle (3) fixe équipé d'une lumière de glissement (17) afin de permettre à l'élément flexible, déformable, mobile d'entraînement (14) de l'objet à distribuer d'être déplacé en translation à l'intérieur de cette lumière de guidage (17).

18. Dispositif de stockage et de distribution (1) selon l'une quelconque des revendications 1 à 16, **caractérisé en ce que** le conteneur (2) est clos sur sa partie supérieure par un couvercle (3) mobile en translation par rapport au conteneur (2).

19. Dispositif de stockage et de distribution (1) selon la revendication 18 **caractérisé en ce qu'**il est muni d'un système de rappel du couvercle en position initiale, situé entre le couvercle (3) et le conteneur (2).

20. Dispositif de stockage et de distribution (1) selon la revendication 18, **caractérisé en ce que** le couvercle (3) mobile en translation par rapport au conteneur (2) comporte sur sa surface d'extrémité supérieure un bossage (4) permettant à l'utilisateur de faire coulisser le couvercle (3) par rapport au conteneur (2).

21. Dispositif de stockage et de distribution (1) selon l'une quelconque des revendications 1 à 16, **caractérisé en ce que** le conteneur (2) est clos sur sa partie supérieure par un couvercle (3) mobile en rotation par rapport au conteneur (2).

22. Dispositif de stockage et de distribution (1) selon la revendication 21, **caractérisé en ce que** le couvercle (3) est articulé en totalité, l'axe de rotation (18) étant placé sur une des extrémité du conteneur (2), coté ouverture de distribution (9).

23. Dispositif de stockage et de distribution (1) selon la revendication 21, **caractérisé en ce que** le couvercle (3) est articulé en partie, une partie du couvercle (3) étant fixe par rapport au conteneur (2), et l'autre partie étant en rotation par rapport à ce conteneur (2), l'axe de rotation (18) étant placé en un lieu compris entre les deux extrémités du couvercle (3).

24. Dispositif de stockage et de distribution (1) selon l'une quelconque des revendications 1 à 23, **caractérisé en ce que** le couvercle (3) comporte sur sa face interne une nervure (15) afin de maintenir l'élément flexible, déformable, mobile d'entraînement (14) en contact avec la face supérieure de l'objet à distribuer.

25. Dispositif de stockage et de distribution (1) selon la revendication 18, **caractérisé en ce que** le couvercle (3) est assemblé sur le conteneur (2) par un système de clipsage et de coulissage par l'intermédiaire de rails avec butée, permettant de solidariser le couvercle (3) et le conteneur (2) sans pour autant restreindre la capacité du couvercle (3) de translater sur le conteneur (2).

26. Dispositif de stockage et de distribution (1) selon la revendication 17, **caractérisé en ce que** le couvercle (3) fixe est assemblé sur le conteneur (2) par un système de clipsage.

27. Dispositif de stockage et de distribution (1) selon l'une quelconque des revendications 1 à 26, **caractérisé en ce que** le dispositif de stockage et de distribution (1) comporte une ouverture de distribution (9) permettant le passage unité par unité des objets à distribuer (8), de type fente dont les bords forment des lèvres ouvertes.

28. Dispositif de stockage et de distribution (1) selon la revendication 27, **caractérisé en ce que** les lèvres de l'ouverture de distribution (9) présentent la forme d'un chanfrein, d'un biseau, ou d'un décalage de l'une des lèvres par rapport à l'autre.

29. Dispositif de stockage et de distribution (1) selon l'une quelconque des revendications 1 à 28, **caractérisé en ce que** l'ouverture de distribution (9) de type fente est soit partie intégrante du conteneur (2), soit partie intégrante du couvercle (3), ou se forme à l'intersection du conteneur (2) et couvercle (3), lors de leur assemblage.

30. Dispositif de stockage et de distribution (1) selon l'une quelconque des revendications 1 à 29, **caractérisé en ce que** l'ouverture de distribution (9) a capacité à s'occulter grâce à un volet flexible.

31. Dispositif de stockage et de distribution (1) selon l'une quelconque des revendications 1 à 30, **caractérisé en ce que** le conteneur (2) et le couvercle (3) sont réalisés dans les matériaux polymères non exclusivement choisis parmi le groupe de matériaux composés de polymères thermoplastiques, comprenant en particulier les polyoléfines telles que les polyéthylènes, les polypropylènes, les copolymères d'éthylène/propylène et leurs mélanges, les polyamides (PA), les polystyrènes (PS), les copolymères d'acrylonitrile-butadiène-styrène (ABS), les copolymères de styrène-acrylonitrile (SAN), les polyméthacrylates de méthyl (PMMA), les polyéthylènetéréphtalates (PET), les polybutylènetéréphtalates (PBT), les polyacétals (POM), les polychlorures de vinyle (PVC), les polycarbonates (PC).

32. Dispositif de stockage et de distribution (1) selon l'une quelconque des revendications 1 à 31, **caractérisé en ce que** l'élément flexible, déformable, mobile d'entraînement (14) est solidaire de la partie mobile du couvercle (3) quand ledit couvercle (3) est mobile.

33. Dispositif de stockage et de distribution (1) selon la revendication 17, **caractérisé en ce que** l'élément flexible, déformable, mobile d'entraînement (14) est désolidarisé du couvercle (3) quand ledit couvercle (3) est fixe.

34. Dispositif de stockage et de distribution (1) selon la revendication 33, **caractérisé en ce que** l'élément flexible, déformable, mobile d'entraînement (14) est équipé d'un sabot coulissant (16) dans la lumière (17) du couvercle fixe (3).

35. Dispositif de stockage et de distribution (1) selon l'une quelconque des revendications 32 à 34, **caractérisé en ce que** l'élément flexible, déformable, mobile d'entraînement (14) est réalisé avec au moins une matière issue du groupe des matières présentant les caractéristiques propres aux compositions élastomériques.

36. Dispositif de stockage et de distribution (1) selon l'une quelconque des revendications 32 à 35, **caractérisé en ce que** l'élément flexible, déformable, mobile d'entraînement (14) est mono-matériau, et formé d'une seule matière thermoplastique élastomérique formulée, mettant en oeuvre au moins un élastomère thermoplastique et un autre composé thermoplastique rigide, préférentiellement de type oléfinique.

37. Dispositif de stockage et de distribution (1) selon l'une quelconque des revendications 32 à 35, **caractérisé en ce que** l'élément flexible, déformable, mobile d'entraînement (14) est multi-matériaux, et comprend une âme faite d'un type de matériau et une peau faite d'un autre type de matériau de type élastomérique.

38. Dispositif de stockage et de distribution (1) selon les revendications 36 ou 37, **caractérisé en ce que** le ou les matériaux élastomères sont choisis préférentiellement dans le groupe constitué par des élastomères de type caoutchoucs naturels, caoutchouc synthétique, en particulier les caoutchoucs de mono-oléfines, tels que, en particulier, les polymères d'isobutylène/isoprène, éthylène-acétate de vinyle (EVA), éthylène-propylène (EPR), éthylène-propylène-diène (EPDM), éthylène-esters acryliques (EMA-EEA), les polymères fluorés, les caoutchouc de dioléfines, tels que, en particulier, les polybutadiène, les copolymères de butadiène-styrène (SBR), les caoutchoucs à base de produits de condensation tels que, en particulier, les caoutchoucs thermoplastiques polyesters et polyuréthanes, les silicones, les caoutchoucs styréniques, tels que styrène-butadiène-styrène(SBS), les styrène-isoprène-styrène (SIS), les styrene-ethylène butadiene styrene (SEBS) et autres copolymères bloc, mis en oeuvre seuls ou en mélange, formulés ou non.

39. Dispositif de stockage et de distribution (1) selon l'une quelconque des revendications 1 à 38, **caractérisé en ce qu'**un système sécuritaire d'ouverture (5) à l'égard des enfants est associé au dispositif de stockage et de distribution (1) d'objets.

40. Dispositif de stockage et de distribution (1) selon la revendication 39, **caractérisé en ce que** le système sécuritaire d'ouverture (5) à l'égard des enfants est un bouton poussoir faisant partie intégrante du couvercle, et permettant par la combinaison d'une pression verticale maintenue et d'une translation simultanée, de déverrouiller le système sécuritaire et d'assurer le fonctionnement du distributeur.

41. Dispositif de stockage et de distribution (1) selon l'une quelconque des revendications 1 à 40, **caractérisé en ce que** le conteneur (2) et le couvercle (3) sont munis, entre ledit conteneur (2) et ledit couvercle (3), d'un témoin d'ouverture du conteneur avant la première ouverture.

42. Dispositif de stockage et de distribution (1) selon l'une quelconque des revendications 39 à 41, **caractérisé en ce que** le système sécuritaire d'ouverture (5) à l'égard des enfants est constitué par des micro-points sécables, témoin de première ouverture, positionné entre le bouton pressoir et son logement.

43. Dispositif de stockage et de distribution (1) selon l'une quelconque des revendications 1 à 42, **caractérisé en ce que** ledit dispositif de stockage et de distribution (1) est rendu dessicatif par l'un au moins des moyens qui consistent en un revêtement interne du fond du conteneur (2) et/ou de la surface interne de la paroi du conteneur (2) et/ou du couvercle (3) au moyen d'une composition polymère thermoplastique dessicative, en l'introduction d'un insert dans ledit dispositif de stockage et de distribution (1), en le revêtement de l'élément flexible, déformable, mobile d'entraînement (14) au moyen d'une composition élastomérique dessicative et/ou en l'introduction d'un matériau dessicatif placé dans un logement particulier sur la surface interne du conteneur (2) et/ou du couvercle (3).

44. Utilisation du dispositif de stockage et de distribution (1) selon les revendications 1 à 43 pour la distribution d'objets en forme de feuilles, plaquettes, bandelettes ou lamelles minces tels que des « strip » tests en forme de bandelettes ou lamelles rigides utilisées par exemple à des fins de diagnostic ou de contrôle dans le domaine médical ou d'objets à distribuer de forme carré ou rectangulaire pouvant également se présenter sous la forme de feuille mince, soit seuls ou avec leur emballage protecteur, en particulier des pansements, des patchs transdermiques, des produits alimentaires en particulier des chewing-gums.

## Claims

1. Storage and distribution device (1), of box type, for objects in the form of thin sheets, packs, strips, or lamellae, of flat geometry, in particular substantially square, rectangular, polygonal, circular, elliptical, permitting distribution unit by unit of the said objects to be distributed (8), including:
- a storage means of container type (2) with a lid (3), containing the objects to be distributed (8), stored stacked one on the other on a storage surface (7), the said storage means comprising a surface (10) for guiding the objects to be distributed (8) and a distribution orifice (9) permitting passage of the objects to be distributed (8) unit by unit,
- and a distribution means
**characterised by** the fact that the said distribution means is formed of a flexible, deformable, moveable driving element (14), contact of one of the ends of which with the upper face of the object to be distributed (8) is a transversal surface to surface contact along at least one tangential line, with a static coefficient of friction greater than the static coefficient of friction existing between two facing surfaces of two contiguous objects to be distributed (8), and the longitudinal section of which is S-shaped or almost U-shaped.

2. Storage and distribution device (1) as described in claim 1, **characterised by** the fact that the flexible, deformable, moveable driving element (14) has the form of a finger of circular, elliptical, or polygonal section, the downward end of which is provided with a transversal bar perpendicularly to its longitudinal axis, providing the transversal surface to surface contact along at least one tangential line with the object to be distributed (8).

3. Storage and distribution device (1) as described in claim 2, **characterised by** the fact that the flexible, deformable, moveable driving element (14) is a thin lamella, of constant width along the whole length of the said flexible element, or becoming uniformly or non-uniformly narrower.

4. Storage and distribution device (1) as described in any one of claims 1 to 3, **characterised by** the fact that the flexible, deformable, moveable driving element (14) preferably has a triangular, rectangular, or T-shaped form.

5. Storage and distribution device (1) as described in claim 4, **characterised by** the fact that when the flexible, deformable, moveable driving element (14) has a rectangular shape, its width is at most equal to a value very slightly smaller than the width L of the container.

6. Storage and distribution device (1) as described in claim 4, **characterised by** the fact that when the flexible, deformable, moveable driving element (14) has a triangular shape, the large base of the triangle forms the transversal contact with the object to be extracted.

7. Storage and distribution device (1) as described in claim 4, **characterised by** the fact that when the flexible, deformable, moveable driving element (14) is T-shaped, the transversal bar of the "T" forms the transversal contact with the object to be distributed, and this bar has a length at most equal to a value very slightly smaller than the width L of the container.

8. Storage and distribution device (1) as described in any one of claims 1 to 7, **characterised by** the fact that the surface of transversal contact with the object to be distributed has a length of between 0.25 times the width L of the container and a length very slightly smaller than the said width L of the container.

9. Storage and distribution device (1) as described in claim 8, **characterised by** the fact that the length is preferably between 0.6 L and a length very slightly smaller than the said width L of the container.

10. Storage and distribution device (1) as described in any one of claims 1 to 9, **characterised by** the fact that the storage means of container type (2) includes a base (6) formed by combination of two contiguous surfaces, one being the storage surface (7) and the other being the guiding surface (10).

11. Storage and distribution device (1) as described in any one of claims 1 to 10, **characterised by** the fact that the storage surface (7), forming a support surface on which the objects to be distributed (8) are stacked, is a generally flat inclined surface forming an acute angle with the plane formed by the lid (3) and is contiguous with the guiding surface (10).

12. Storage and distribution device (1) as described in any one of claims 1 to 10, **characterised by** the fact that the storage surface (7), forming a support surface on which the objects to be distributed (8) are stacked, is a surface parallel with the plane of the lid (3) and is contiguous with the guiding surface (10).

13. Storage and distribution device (1) as described in any one of claims 1 to 12, **characterised by** the fact that the guiding surface (10) is a developable curvilinear surface, forming an outlet ramp and emerging at a distribution opening (9).

14. Storage and distribution device (1) as described in any one of claims 1 to 12, **characterised by** the fact that the guiding surface (10) is formed of one or more planes inclined relative to the plane formed by the lid (2) at the distribution opening (9), so as to form a funnel permitting guiding of the object to be distributed.

15. Storage and distribution device (1) as described in any one of claims 1 to 14, **characterised by** the fact that the container (2) includes two substantially parallel vertical walls (11) and (12) permitting partial closure of the container (2) and lateral positioning of the objects to be distributed (8) and also a third vertical wall (13) substantially perpendicular to the two vertical walls (11) and (12), positioned on the side opposite to the distribution opening (9) on which the objects to be distributed (8) rest with their edges.

16. Storage and distribution device (1) as described in any one of claims 1 to 14, **characterised by** the fact that the two walls (11) and (12) are convergent.

17. Storage and distribution device (1) as described in any one of claims 1 to 16, **characterised by** the fact that the container (2) is closed at its upper part by a fixed lid (3) provided with a slide aperture (17) in order to allow the flexible, deformable, moveable element (14) for driving the object to be distributed to be displaced in translation within this guiding aperture (17).

18. Storage and distribution device (1) as described in any one of claims 1 to 16, **characterised by** the fact that the container (2) is closed at its upper part by a lid (3) moveable in translation relative to the container (2).

19. Storage and distribution device (1) as described in claim 18, **characterised by** the fact that it is provided with a system for returning the lid into the initial position, situated between the lid (3) and the container (2).

20. Storage and distribution device (1) as described in claim 18, **characterised by** the fact that the lid (3) moveable in translation relative to the container (2) includes on its upper end surface a boss (4) allowing the user to slide the lid (3) relative to the container (2).

21. Storage and distribution device (1) as described in any one of claims 1 to 16, **characterised by** the fact that the container (2) is closed at its upper part by a lid (3) which is moveable in rotation relative to the container (2).

22. Storage and distribution device (1) as described in claim 21, **characterised by** the fact that the lid (3) is hinged in its totality, the axis of rotation (18) being positioned on one of the ends of the container (2) on the distribution opening (9) side.

23. Storage and distribution device (1) as described in claim 21, **characterised by** the fact that the lid (3) is partially hinged, a part of the lid (3) being fixed relative to the container (2), and the other part being in rotation relative to this container (2), the axis of rotation (18) being positioned in a location between the two ends of the lid (3).

24. Storage and distribution device (1) as described in any one of claims 1 to 23, **characterised by** the fact that the lid (3) includes a rib (15) on its inside face in order to keep the flexible, deformable, moveable driving element (14) in contact with the upper face of the object to be distributed.

25. Storage and distribution device (1) as described in claim 18, **characterised by** the fact that the lid (3) is assembled with the container (2) by a snap-on and sliding system by means of rails with a stop, permitting attachment of the lid (3) to the container (2) without thereby restricting the capacity of the lid (3) to translate on the container (2).

26. Storage and distribution device (1) as described in claim 17, **characterised by** the fact that the fixed lid (3) is assembled with the container (2) by a snap-on system.

27. Storage and distribution device (1) as described in any one of claims 1 to 26, **characterised by** the fact that the storage and distribution device (1) includes a distribution opening (9) permitting passage unit by unit of the objects to be distributed (8), of the type of a slot the edges of which form open lips.

28. Storage and distribution device (1) as described in claim 27, **characterised by** the fact that the lips of the distribution opening (9) have the form of a chamfer, bevel, or offset of one of the lips relative to the other.

29. Storage and distribution device (1) as described in any one of claims 1 to 28, **characterised by** the fact that the distribution opening (9) of slot type is either an integral part of the container (2), or an integral part of the lid (3), or is formed at the intersection of the container (2) and the lid (3), when they are assembled.

30. Storage and distribution device (1) as described in any one of claims 1 to 29, **characterised by** the fact that the distribution opening (9) has the ability to be occulted by means of a flexible shutter.

31. Storage and distribution device (1) as described in any one of claims 1 to 30, **characterised by** the fact that the container (2) and the lid (3) are made of polymer materials not exclusively selected from the group of materials composed of thermoplastic polymers, comprising in particular polyolefins such as polyethylenes, polypropylenes, ethylene/propylene copolymers and their mixtures, polyamides (PA), polystyrenes (PS), acrylonitrile-butadiene-styrene copolymers (ABS), styrene-acrylonitrile copolymers (SAN), methyl polymethacrylates (PMMA), polyethyleneterephthalates (PET), polybutyleneterephthalates (PBT), polyacetals (POM), polyvinyl chlorides (PVC) and polycarbonates (PC).

32. Storage and distribution device (1) as described in any one of claims 1 to 31, **characterised by** the fact that the flexible, deformable, moveable driving element (14) is attached to the moveable part of the lid (3) when the said lid (3) is moveable.

33. Storage and distribution device (1) as described in claim 17, **characterised by** the fact that the flexible, deformable, moveable driving element (14) is detached from the lid (3) when the said lid (3) is fixed.

34. Storage and distribution device (1) as described in claim 33, **characterised by** the fact that the flexible, deformable, moveable driving element (14) is provided with a shoe (16) sliding in the aperture (17) in the fixed lid (3).

35. Storage and distribution device (1) as described in any one of claims 32 to 34, **characterised by** the fact that the flexible, deformable, moveable driving element (14) is made with at least one material from the group of materials having the characteristics of elastomeric compositions.

36. Storage and distribution device (1) as described in any one of claims 32 to 35, **characterised by** the fact that the flexible, deformable, moveable driving element (14) is monomaterial, and formed of a single formulated elastomeric thermoplastic material employing a thermoplastic elastomer and another rigid thermoplastic compound, preferably of olefin type.

37. Storage and distribution device (1) as described in any one of claims 32 to 35, **characterised by** the fact that the flexible, deformable, moveable driving element (14) is multimaterial, and comprises a core made of one type of material and a skin made of another type of material of elastomeric type.

38. Storage and distribution device (1) as described in claims 36 or 37, **characterised by** the fact that the elastomeric material or materials are preferably selected from the group formed of elastomers of the type of natural rubbers, synthetic rubber, in particular monoolefin rubbers, such as, in particular, polymers of isobutylene/isoprene, ethylene vinyl acetate (EVA), ethylene propylene (EPR), ethylene propylene diene, (EPDM), acrylic ethylene esters (EMA-EEA), fluorinated polymers, diolefin rubber, such as, in particular, polybutadiene, butadiene-styrene copolymers (SBR), rubbers based on condensation products such as, in particular, thermoplastic polyester and polyurethane rubbers, silicones, styrene rubbers, such as styrenebutadiene-styrene (SBS), styrene-isoprene-styrene (SIS), styrene-ethylene butadiene styrene (SEBS) and other block copolymers, employed alone or in mixture, formulated or otherwise.

39. Storage and distribution device (1) as described in any one of claims 1 to 38, **characterised by** the fact that a secure opening system (5) relative to children is associated with the device for storage and distribution of objects (1).

40. Storage and distribution device (1) as described in claim 39, **characterised by** the fact that the secure opening system (5) relative to children is a push button forming an integral part of the lid, and permitting, by combination of a maintained vertical pressure and simultaneous translation, unlocking of the secure system and operation of the distributor.

41. Storage and distribution device (1) as described in any one of claims 1 to 40, **characterised by** the fact that the container (2) and the lid (3) are provided, between the said container (2) and the said lid (3), with a revealer of opening the container before the first opening.

42. Storage and distribution device (1) as described in any one of claims 39 to 41, **characterised by** the fact that the secure opening system (5) relative to children consists of dryable micro dots, revealing first opening, positioned between the press button and its housing.

43. Storage and distribution device (1) as described in any one of claims 1 to 42, **characterised by** the fact that the said storage and distribution device (1) is rendered desiccative by at least one of the means which consist of internal coating of the base of the container (2) and/or of the inside surface of the wall of the container (2) and/or of the lid (3) with a desiccative thermoplastic polymer composition, of introduction of an insert into the said storage and distribution device (1), of coating the flexible, deformable, moveable driving element (14) with a desiccative elastomeric composition and/or of introduction of a desiccative material positioned in a special housing on the inside surface of the container (2) and/or of the lid (3).

44. Use of the storage and distribution device (1) as described in claims 1 to 43 for the distribution of objects in the form of thin sheets, packs, strips, or lamellae, such as test "strips" in the form of rigid strips or lamellae used for example for diagnostic or monitoring purposes in the medical field or objects to be distributed in square or rectangular form which may also be in the form of thin sheet, either alone or with their protective packaging, in particular dressings, transdermic patches, food products in particular chewing-gum.

## Patentansprüche

1. Gehäuseartige Vorrichtung zur Lagerung und Abgabe (1) für Gegenstände in Form von dünnen Folien, Plättchen, Streifen oder Lamellen, mit einer ebenen Geometrie, die insbesondere weitestgehend quadratisch, rechteckig, vieleckig, kreisförmig, elliptisch ist, welche die Abgabe der abzugebenden Gegenstände (8) Einheit für Einheit ermöglicht, umfassend
- ein Lagerungsmittel von der Art eines Behälters (2) mit einem Deckel (3), welches die abzugebenden Gegenstände (8) enthält, die aufeinander gestapelt auf einer Lagerungsfläche (7) gelagert sind, wobei das Lagerungsmittel eine Fläche zur Führung (10) der abzugebenden Gegenstände (8) und eine Abgabeöffnung (9), welche die abzugebenden Gegenstände (8) Einheit für Einheit durchlässt, aufweist, und
- ein Abgabemittel,
**dadurch gekennzeichnet, dass** das Abgabemittel durch ein flexibles, verformbares, bewegliches Mitnahmeelement (14) gebildet ist, bei dem der Kontakt eines seiner Enden mit der Oberseite des abzugebenden Gegenstandes (8) ein Querkontakt Fläche gegen Fläche gemäß mindestens einer tangentialen Linie mit einem statischen Reibungskoeffizienten, der höher ist als der statische Reibungskoeffizient, der zwischen zwei gegenüberliegenden Flächen zweier benachbarter abzugebender Gegenstände (8) besteht, ist, und dessen Längsschnitt S- oder nahezu U-förmig ist.

2. Lagerungs- und Abgabevorrichtung (1) nach Anspruch 1,
**dadurch gekennzeichnet, dass** das flexible, verformbare, bewegliche Mitnahmeelement (14) die Form eines Fingers mit einem kreisförmigen, elliptischen oder vieleckigen Querschnitt aufweist, dessen abwärtiges Ende mit einem senkrecht zu seiner Längsachse verlaufenden Quersteg versehen ist, der den Querkontakt Fläche gegen Fläche gemäß mindestens einer tangentialen Linie mit dem abzugebenden Gegenstand (8) sicherstellt.

3. Lagerungs- und Abgabevorrichtung (1) nach Anspruch 2,
**dadurch gekennzeichnet, dass** es sich bei dem flexiblen, verformbaren, beweglichen Mitnahmeelement (14) um ein dünnes, eine konstante Breite auf der gesamten Länge des flexiblen Elements aufweisendes oder regelmäßig oder unregelmäßig verengtes Blatt handelt.

4. Lagerungs- und Abgabevorrichtung (1) nach einem der Ansprüche 1 bis 3, **dadurch gekennzeichnet, dass** das flexible, verformbare, bewegliche Mitnahmeelement (14) vorzugsweise dreieckig, rechteckig oder T-förmig ausgebildet ist.

5. Lagerungs- und Abgabevorrichtung (1) nach Anspruch 4,
**dadurch gekennzeichnet, dass**, wenn das flexible, verformbare, bewegliche Mitnahmeelement (14) rechteckig ausgebildet ist, dessen Breite höchstens gleich einem Wert ist, der sehr geringfügig geringer ist als die Breite L des Behälters.

6. Lagerungs- und Abgabevorrichtung (1) nach Anspruch 4,
**dadurch gekennzeichnet, dass**, wenn das flexible, verformbare, bewegliche Mitnahmeelement (14) dreieckig ausgebildet ist, die große Basis des Dreiecks den Querkontakt mit dem herauszunehmenden Gegenstand bildet.

7. Lagerungs- und Abgabevorrichtung (1) nach Anspruch 4,
**dadurch gekennzeichnet, dass**, wenn das flexible, verformbare, bewegliche Mitnahmeelement (14) T-förmig ausgebildet ist, der Quersteg des T den Querkontakt mit dem abzugebenden Gegenstand bildet und dieser Steg eine Länge aufweist, die höchstens gleich einem Wert ist, der sehr geringfügig geringer ist als die Breite L des Behälters.

8. Lagerungs- und Abgabevorrichtung (1) nach einem der Ansprüche 1 bis 7, **dadurch gekennzeichnet, dass** die Fläche des Querkontakts mit dem abzugebenden Gegenstand eine Länge aufweist, die zwischen 0,25 Mal die Breite L des Behälters und einer Länge, die sehr geringfügig geringer als die Breite L des Behälters ist, liegt.

9. Lagerungs- und Abgabevorrichtung (1) nach Anspruch 8,
**dadurch gekennzeichnet, dass** die Länge vorzugsweise zwischen 0,6 L und einer Länge, die sehr geringfügig geringer als die Breite L des Behälters ist, liegt.

10. Lagerungs- und Abgabevorrichtung (1) nach einem der Ansprüche 1 bis 9, **dadurch gekennzeichnet, dass** das Lagerungsmittel von der Art eines Behälters (2) einen Boden (6) aufweist, der durch die Kombination zweier benachbarter Flächen gebildet ist, wobei die eine die Lagerungsfläche (7) ist und die andere die Führungsfläche (10) ist.

11. Lagerungs- und Abgabevorrichtung (1) nach einem der Ansprüche 1 bis 10, **dadurch gekennzeichnet, dass** die Lagerungsfläche (7), die eine Auflagefläche bildet, auf der die abzugebenden Gegenstände (8) gestapelt sind, eine in der Regel ebene, geneigte Fläche, die mit der durch den Deckel (3) gebildeten Ebene einen spitzen Winkel bildet, ist und mit der Führungsfläche (10) benachbart ist.

12. Lagerungs- und Abgabevorrichtung (1) nach einem der Ansprüche 1 bis 10, **dadurch gekennzeichnet, dass** die Lagerungsfläche (7), die eine Auflagefläche bildet, auf der die abzugebenden Gegenstände (8) gestapelt sind, eine Fläche, die parallel zu der Ebene des Deckels (3) verläuft, ist und mit der Führungsfläche (10) benachbart ist.

13. Lagerungs- und Abgabevorrichtung (1) nach einem der Ansprüche 1 bis 12, **dadurch gekennzeichnet, dass** die Führungsfläche (10) eine gekrümmte abwickelbare Fläche ist, die eine Austrittsrampe bildet und in die Abgabeöffnung (9) mündet.

14. Lagerungs- und Abgabevorrichtung (1) nach einem der Ansprüche 1 bis 12, **dadurch gekennzeichnet, dass** die Führungsfläche (10) durch eine oder mehrere Ebenen gebildet ist, die in Bezug auf die durch den Deckel (2) an der Abgabeöffnung (9) gebildete Ebene derart geneigt sind, dass ein Trichter gebildet wird, der die Führung des abzugebenden Gegenstandes ermöglicht.

15. Lagerungs- und Abgabevorrichtung (1) nach einem der Ansprüche 1 bis 14, **dadurch gekennzeichnet, dass** der Behälter (2) zwei vertikale, weitestgehend parallele Wände (11) und (12), die es ermöglichen, den Behälter (2) teilweise zu schließen und die abzugebenden Gegenstände (8) seitlich zu positionieren, sowie eine dritte vertikale, weitestgehend senkrecht zu den zwei vertikalen Wänden (11) und (12) verlaufende Wand (13), die auf der der Abgabeöffnung (9) entgegengesetzten Seite, auf der die abzugebenden Gegenstände (8) mit ihrem Rand zur Auflage kommen, positioniert ist, aufweist.

16. Lagerungs- und Abgabevorrichtung (1) nach einem der Ansprüche 1 bis 14, **dadurch gekennzeichnet, dass** die zwei Wände (11) und (12) konvergieren.

17. Lagerungs- und Abgabevorrichtung (1) nach einem der Ansprüche 1 bis 16, **dadurch gekennzeichnet, dass** der Behälter (2) in seinem oberen Bereich mit einem festen Deckel (3) verschlossen ist, der mit einer Gleitöffnung (17) versehen ist, damit das flexible, verformbare, bewegliche Element (14) zur Mitnahme des abzugebenden Gegenstandes innerhalb dieser Führungsöffnung (17) verschoben werden kann.

18. Lagerungs- und Abgabevorrichtung (1) nach einem der Ansprüche 1 bis 16, **dadurch gekennzeichnet, dass** der Behälter (2) in seinem oberen Bereich mit einem Deckel (3) verschlossen ist, der in Bezug auf den Behälter (2) verschiebbar ist.

19. Lagerungs- und Abgabevorrichtung (1) nach Anspruch 18,
**dadurch gekennzeichnet, dass** sie mit einem System zur Rückführung des Deckels in die ursprüngliche Stellung versehen ist, welches zwischen dem Deckel (3) und dem Behälter (2) angeordnet ist.

20. Lagerungs- und Abgabevorrichtung (1) nach Anspruch 18,
**dadurch gekennzeichnet, dass** der in Bezug auf den Behälter (2) verschiebbare Deckel (3) auf seiner oberen Endfläche eine Nocke (4) aufweist, mit der der Benutzer den Deckel (3) in Bezug auf den Behälter (2) verschieben kann.

21. Lagerungs- und Abgabevorrichtung (1) nach einem der Ansprüche 1 bis 16, **dadurch gekennzeichnet, dass** der Behälter (2) in seinem oberen Bereich mit einem Deckel (3) verschlossen ist, der in Bezug auf den Behälter (2) drehbeweglich ist.

22. Lagerungs- und Abgabevorrichtung (1) nach Anspruch 21,
**dadurch gekennzeichnet, dass** der Deckel (3) vollständig gelenkig ist, wobei die Drehachse (18) an einem der Enden des Behälters (2) auf der Seite der Abgabenöffnung (9) angeordnet ist.

23. Lagerungs- und Abgabevorrichtung (1) nach Anspruch 21,
**dadurch gekennzeichnet, dass** der Deckel (3) teilweise gelenkig ist, wobei ein Teil des Deckels (3) in Bezug auf den Behälter (2) fest ist und der andere Teil sich in Bezug auf diesen Behälter (2) dreht, wobei die Drehachse (18) an einem Ort zwischen den beiden Enden des Deckels (3) angeordnet ist.

24. Lagerungs- und Abgabevorrichtung (1) nach einem der Ansprüche 1 bis 23, **dadurch gekennzeichnet, dass** der Deckel (3) auf seiner Innenseite eine Rippe (15) aufweist, um das flexible, verformbare, bewegliche Mitnahmeelement (14) in Kontakt mit der Oberseite des abzugebenden Gegenstandes zu halten.

25. Lagerungs- und Abgabevorrichtung (1) nach Anspruch 18,
**dadurch gekennzeichnet, dass** der Deckel (3) an dem Behälter (2) durch ein Clip- und Verschiebesystem über Schienen mit Anschlag montiert ist, mit dem der Deckel (3) und der Behälter (2) verbunden werden können, ohne die Fähigkeit des Deckels (3), sich auf dem Behälter (2) zu verschieben, einzuschränken.

26. Lagerungs- und Abgabevorrichtung (1) nach Anspruch 17,
**dadurch gekennzeichnet, dass** der feste Deckel (3) an dem Behälter (2) durch ein Clip-System montiert ist.

27. Lagerungs- und Abgabevorrichtung (1) nach einem der Ansprüche 1 bis 26, **dadurch gekennzeichnet, dass** die Lagerungs- und Abgabevorrichtung (1) eine die abzugebenden Gegenstände (8) Einheit für Einheit durchlassende Abgabeöffnung (9) von der Art eines Schlitzes, dessen Ränder offene Lippen bilden, aufweist.

28. Lagerungs- und Abgabevorrichtung (1) nach Anspruch 27,
**dadurch gekennzeichnet, dass** die Lippen der Abgabeöffnung (9) die Form einer Fase, einer Schräge oder eines Versatzes der Lippen zueinander aufweisen.

29. Lagerungs- und Abgabevorrichtung (1) nach einem der Ansprüche 1 bis 28, **dadurch gekennzeichnet, dass** die schlitzartige Abgabeöffnung (9) entweder Bestandteil des Behälters (2) oder Bestandteil des Deckels (3) ist oder sich an der Schnittstelle zwischen dem Behälter (2) und dem Deckel (3) bei deren Zusammenbau bildet.

30. Lagerungs- und Abgabevorrichtung (1) nach einem der Ansprüche 1 bis 29, **dadurch gekennzeichnet, dass** die Abgabeöffnung (9) in der Lage ist, sich durch eine flexible Klappe zu verdecken.

31. Lagerungs- und Abgabevorrichtung (1) nach einem der Ansprüche 1 bis 30, **dadurch gekennzeichnet, dass** der Behälter (2) und der Deckel (3) aus den Polymermaterialien hergestellt sind, die nicht ausschließlich aus der Gruppe von Materialien gewählt sind, die aus thermoplastischen Polymeren bestehen, umfassend insbesondere die Polyolefine wie die Polyethylene, die Polypropylene, die Ethylen-Propylen-Copolymere und deren Gemische, die Polyamide (PA), die Polystyrene (PS), die Acrylnitril-Butadien-Styren-Copolymere (ABS), die Styren-Acrylnitril-Copolymere (SAN), die Methylpolymethacrylate (PMMA), die Polyethylenterephtalate (PET), die Polybutylenterephtalate (PBT), die Polyacetale (POM), die Polyvinylchloride (PVC), die Polycarbonate (PC).

32. Lagerungs- und Abgabevorrichtung (1) nach einem der Ansprüche 1 bis 31, **dadurch gekennzeichnet, dass** das flexible, verformbare, bewegliche Mitnahmeelement (14) mit dem beweglichen Teil des Deckels (3) verbunden ist, wenn der Deckel (3) beweglich ist.

33. Lagerungs- und Abgabevorrichtung (1) nach Anspruch 17,
**dadurch gekennzeichnet, dass** das flexible, verformbare, bewegliche Mitnahmeelement (14) von dem Deckel (3) gelöst ist, wenn der Deckel (3) fest ist.

34. Lagerungs- und Abgabevorrichtung (1) nach Anspruch 33,
**dadurch gekennzeichnet, dass** das flexible, verformbare, bewegliche Mitnahmeelement (14) in der Öffnung (17) des festen Deckels (3) mit einem verschieblichen Klotz (16) versehen ist.

35. Lagerungs- und Abgabevorrichtung (1) nach einem der Ansprüche 32 bis 34, **dadurch gekennzeichnet, dass** das flexible, verformbare, bewegliche Mitnahmeelement (14) mindestens aus einem Material aus der Gruppe der Materialien mit den Eigenschaften, die den Elastomerverbindungen eigen sind, hergestellt ist.

36. Lagerungs- und Abgabevorrichtung (1) nach einem der Ansprüche 32 bis 35, **dadurch gekennzeichnet, dass** das flexible, verformbare, bewegliche Mitnahmeelement (14) ein Monomaterial ist und aus einem einzigen formulierten thermoplastischen Elastomermaterial besteht, bei dem mindestens ein thermoplastisches Elastomer und eine weitere starre thermoplastische, vorzugsweise olefinartige Verbindung eingesetzt werden.

37. Lagerungs- und Abgabevorrichtung (1) nach einem der Ansprüche 32 bis 35, **dadurch gekennzeichnet, dass** das flexible, verformbare, bewegliche Mitnahmeelement (14) ein Multi-Material ist und einen Kern aus einem Materialtyp sowie eine Haut aus einem anderen elastomerartigen Material umfasst.

38. Lagerungs- und Abgabevorrichtung (1) nach Ansprüchen 36 oder 37, **dadurch gekennzeichnet, dass** das bzw. die Elastomermaterialien vorzugsweise aus der Gruppe gewählt werden, bestehend aus Elastomeren von der Art natürlicher Kautschuke, synthetischen Kautschuks, insbesondere die Monoolefin-Kautschuke wie insbesondere die Polymere aus Isobutylen-Isopren, Vinylethylenacetat (EVA), Ethylen-Propylen (EPR), Ethylen-Propylen-Dien (EPDM), Ethylen-Acrylestere (EMA-EEA), die Fluorpolymere, die Diolefin-Kautschuke wie insbesondere die Polybutadiene, Butadien-Styren-Copolymere (SBR), die Kautschuke auf Basis von Kondensationsprodukten wie insbesondere die thermoplastischen Polyester- und Polyurethan-Kautschuke, die Silikone, die Styrenkautschuke wie Styren-Butadien-Styren (SBS), die Styen-Isopren-Styrene (SIS), die Styren-Ethylen-Butadien-Styrene (SEBS) und weitere Blockcopolymere, die alleine oder gemischt, formuliert oder nicht formuliert eingesetzt werden.

39. Lagerungs- und Abgabevorrichtung (1) nach einem der Ansprüche 1 bis 38, **dadurch gekennzeichnet, dass** ein kindersicheres Öffnungssystem (5) der Vorrichtung zur Lagerung und Abgabe (1) von Gegenständen zugeordnet ist.

40. Lagerungs- und Abgabevorrichtung (1) nach Anspruch 39,
**dadurch gekennzeichnet, dass** es sich bei dem kindersicheren Öffnungssystem (5) um einen Druckknopf handelt, der Bestandteil des Deckels ist und es ermöglicht, durch eine Kombination aus einem vertikalen anhaltenden Druck und einer gleichzeitigen Verschiebung das Sicherheitssystem zu entriegeln und den Betrieb der Abgabevorrichtung sicherzustellen.

41. Lagerungs- und Abgabevorrichtung (1) nach einem der Ansprüche 1 bis 40, **dadurch gekennzeichnet, dass** der Behälter (2) und der Deckel (3) zwischen dem Behälter (2) und dem Deckel (3) mit einem Indikator der Öffnung des Behälters vor der ersten Öffnung versehen sind.

42. Lagerungs- und Abgabevorrichtung (1) nach einem der Ansprüche 39 bis 41, **dadurch gekennzeichnet, dass** das kindersichere Öffnungssystem (5) durch abtrennbare Mikropunkte als Indikator der Erstöffnung gebildet ist, der zwischen dem Druckknopf und seiner Aufnahme angeordnet ist.

43. Lagerungs- und Abgabevorrichtung (1) nach einem der Ansprüche 1 bis 42, **dadurch gekennzeichnet, dass** die Lagerungs- und Abgabevorrichtung (1) durch mindestens eines der Mittel trocknend wird, die in einer inneren Beschichtung des Bodens des Behälters (2) und/oder der Innenfläche der Wand der Behälters (2) und/oder des Deckels (3) mittels einer thermoplastischen trocknenden Polymerverbindung, in der Einführung einer Einlage in die Lagerungs- und Abgabevorrichtung (1), in der Beschichtung des flexiblen, verformbaren, beweglichen Mitnahmeelements (14) mittels einer trocknenden Elastomerverbindung und/oder in der Einführung eines Trocknungsmaterials, das in einer besonderen Aufnahme auf der Innenfläche des Behälters (2) und/oder des Deckels (3) eingesetzt ist, bestehen.

44. Verwendung der Lagerungs- und Abgabevorrichtung (1) nach einem der Ansprüche 1 bis 43 zur Abgabe von Gegenständen in Form von dünnen Folien, Plättchen, Streifen oder Lamellen wie Teststrips in Form von starren Streifen oder Lamellen, die beispielweise zu Diagnose- oder Kontrollzwecken in der Medizin eingesetzt werden, oder von abzugebenden Gegenständen, die quadratisch oder rechteckig ausgebildet sind, ebenfalls als dünne Folie alleine oder mit ihrer Schutzverpackung ausgestaltet sein können, insbesondere Pflaster, transdermale Hautpatches, Lebensmittel, insbesondere Kaugummis.
